# EUROPEAN PATENT SPECIFICATION

(11) **EP 2 533 770 B1**
(45) Date of publication and mention of the grant of the patent: **04.10.2017**
(21) Application number: 11742930.8
(22) Date of filing: 12.02.2011
(51) Int. Cl.: A23L 29/256, A23L 29/275, A23P 20/10, A61K 47/44, A61K 47/36

(54) **ENTERIC COATING COMPOSITIONS AND METHOD OF MAKING THE SAME**
ENTERALE BESCHICHTUNGSZUSAMMENSETZUNGEN SOWIE VERFAHREN ZU IHRER HERSTELLUNG
COMPOSITIONS D'ENROBAGE ENTÉRIQUE ET PROCÉDÉ DE FABRICATION DE CELUI-CI

(30) Priority: 12.02.2010 US 304224 P
(43) Date of publication of application: 19.12.2012
(73) Proprietor: Sensient Colors LLC, St. Louis, MO 63106 (US)
(72) Inventor: VAN NESS, Eric, H., St. Louis, MO 63122 (US); SCHAD, Beverly, A., Stoughton, WI 53589 (US); RILEY, Thomas, C., St. Charles, MO 63304 (US); CHENG, Brian, K., Chesterfield, MO 63017 (US)
(74) Representative: Stafford, Jonathan Alan Lewis
(86) International application number: PCT/US2011/024663
(87) International publication number: WO 2011/100643

(56) References cited:
- WO-A2-03/001921
- US-A1- 2006 165 778
- US-A1- 2007 059 270
- US-A1- 2007 071 821
- US-A1- 2008 038 362
- US-A1- 2008 057 086
- US-A1- 2009 252 767
- US-B1- 6 183 775

## Description

### BACKGROUND

In many cases it is desirable for pharmaceutical and nutraceutical dosage units to be able to pass through a stomach intact and release their contents upon reaching intestines. This may be particularly desirable when an ingredient(s) of the dosage unit is unstable in the acidic environment of the stomach and where the ingredient(s) is intended for release in the slightly alkaline conditions of the gastrointestinal tract beyond the stomach. Pharmaceutical dosage units have been able to pass through the stomach intact and release their contents upon reaching the intestines by using an enteric coating.

Enteric coating materials are generally acid resistant, thus protecting and preventing a dosage unit from releasing its contents into the stomach. These coatings dissolve or disintegrate in the neutral or mildly alkaline conditions of the gastrointestinal tract beyond the stomach.

US2007/071821A1 describes a formulation for providing an enteric coating material as well as methods of providing and using the material

US2009/0252767A1 describes water dispersible enteric coating formulations of nutraceutical and pharmaceutical dosage forms.

### BRIEF SUMMARY

An enteric coating composition is provided, as described in claim 1. The enteric coating composition comprises 0.01% to 8% ammoniated shellac and 0.01% to 8% polymer selected from the group consisting of alginates and alginic acid, and less than 15% solids, wherein the total content of the resin in the composition selected from shellacs, plant resins and synthetic resins is 0.01% to 10% and the total content of non-resin polymer in the composition is 0.01% to 10%.

In addition, the present disclosure describes a multiple-component system comprising a first component comprising a resin and a second component comprising a polymer. A mixture comprising the first component and the second component may form an enteric coating composition comprising about 0.01 % to about 10% resin and about 0.01 % to about 10% polymer.

In another aspect, the disclosure provides a pharmaceutical or nutraceutical comprising a pharmaceutical agent or nutraceutical agent coated with an enteric coating composition, as described in claim 5. The enteric coating composition comprises 0.01% to 8% resin and 0.01% to 8% alginate, wherein the resin is ammoniated shellac and the enteric coating composition comprises less than 15% solids.

In another aspect, the disclosure provides a method of making a product according to claim 7. The method comprises applying an enteric coating composition to a substrate to form an enteric coating on the substrate. The enteric coating composition comprises 0.01 % to 8% ammoniated shellac and 0.01% to 8% polymer selected from the group consisting of alginates and alginic acid, and less than 15% solids, wherein the total content of the resin in the composition selected from shellacs, plant resins and synthetic resins is 0.01 % to 10% and the total content of non-resin polymer in the composition is 0.01% to 10%.

In another aspect, the disclosure provides a method for coating a substrate comprising loading the substrate into a coating pan and coating the substrate with an enteric coating composition to form an enteric coating, the enteric coating composition comprising 0.01% to about 8% ammoniated shellac and 0.01% to about 8% polymer selected from the group consisting of alginates and alginic acid, and less than 15% solids, wherein the total content of the resin selected from shellacs, plant resins and synthetic resins in the composition is 0.01% to 10% and the total content of non-resin polymer in the composition is 0.01% to 10%.

### BRIEF DESCRIPTION OF THE DRAWINGS

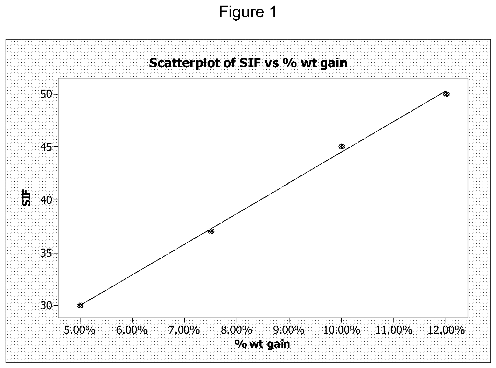

Figure 1 depicts SIF disintegration times (in minutes) of capsules coated with an enteric coating as a function of the weight gain percentage during the coating process.

### DETAILED DESCRIPTION

The present disclosure is not limited in its disclosure to the specific details of construction, arrangement of components, or method steps set forth herein. The compositions and methods disclosed herein are capable of being made, practiced, used, carried out and/or formed in various ways. The phraseology and terminology used herein is for the purpose of description only and should not be regarded as limiting. Ordinal indicators, such as first, second, and third, as used in the description and the claims to refer to various structures or method steps, are not meant to be construed to indicate any specific structures or steps, or any particular order or configuration to such structures or steps. All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification, and no structures shown in the drawings, should be construed as indicating that any non-claimed element is essential to the practice of the disclosed subject matter. The use herein of the terms "including," "comprising," or "having," and variations thereof, is meant to encompass the items listed thereafter and equivalents thereof, as well as additional items. Unless specified or limited otherwise, the terms "mounted," "connected," "supported," and "coupled" and variations thereof encompass both direct and indirect mountings, connections, supports, and couplings. Further, "connected" and "coupled" are not restricted to physical or mechanical connections or couplings.

Recitation of ranges of values herein are merely intended to serve as a shorthand method of referring individually to each separate value falling within the range, unless otherwise indicated herein, and each separate value is incorporated into the specification as if it were individually recited herein. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure. Use of the word "about" to describe a particular recited amount or range of amounts is meant to indicate that values very near to the recited amount are included in that amount, such as values that could or naturally would be accounted for due to manufacturing tolerances, instrument and human error in forming measurements, and the like. All percentages referring to amounts are by weight unless indicated otherwise.

No admission is made that any reference, including any non-patent or patent document cited in this specification, constitutes prior art. In particular, it will be understood that, unless otherwise stated, reference to any document herein does not constitute an admission that any of these documents forms part of the common general knowledge in the art in the United States or in any other country. Any discussion of the references states what their authors assert, and the applicant reserves the right to challenge the accuracy and pertinence of any of the documents cited herein. The present disclosure shall control in the event there are any disparities.

The enteric coatings of the present application may fulfill the needs of the pharmaceutical and dietary supplement markets seeking an innovative coating technology formulated with food approved components. Some embodiments may provide an enteric coating and method of making the same that may produce a delayed release profile that meets the current USP testing method for enteric coated substrates using food and drug approved materials.

In one embodiment, enteric coating compositions are provided that are used to form enteric coatings for, among other things, pharmaceuticals; nutraceuticals; foods, such as, for example, fruits and vegetables; agricultural products, such as, for example, seeds and animal feeds; and industrial products. The enteric coating composition comprises a resin (namely ammoniated shellac), a polymer (namely an alginate), and may comprise a plasticizer, a preservative, a detackifying agent, a lubricant, an emulsifier, a surfactant, a colorant, a flavor, a sweetener, a taste maskant, an opacifier, a buffering agent, an antioxidant, a solvent, and combinations thereof. These components may be water soluble, water insoluble, or water miscible. For example, the enteric coating composition comprises ammoniated shellac), and an alginate which may be a water-soluble, water-insoluble, or water-miscible alginate. At least one of these materials may be approved for food use. When applied to orally ingestible substrates such as pharmaceutical tablets and dietary supplements, the enteric coating composition may form an enteric coating. The enteric coating composition may be applied onto oral dosage substrates, such as pharmaceutical tablets and dietary supplements, to provide a delayed release enteric film.

Examples of resins include, but are not limited to, shellacs, plant resins, and synthetic resins.

Shellac is an exudate of the lac insect and is a natural material that is insoluble in water but soluble in organic solvents including ethanol. The term shellac covers the range of this type of material. As shellac is insoluble in acidic conditions but soluble at higher pH levels (i.e., pH greater than about 6.5) it would appear to be suitable as an enteric coating material. In practice, delayed disintegration and delayed drug release occurs *in vivo* as the shellac coat is typically not soluble in the upper intestine. Shellac commonly does not behave in a typical enteric coating manner and instead behaves more like an erodible coating, dissolving as a function of time rather than of pH.

Examples of shellacs include, but are not limited to, dewaxed bleached shellacs, dewaxed and decolorized shellacs (dewaxed orange shellac), and all USP shellacs. The shellac may be in an aqueous salt form or free acid form. For instance, the shellac may be an aqueous alkali salt of shellac. The shellac may also be an aqueous ammonium salt of shellac. The shellac may be formed out of water and not alcohol. The aqueous shellac may have at least about 5%, at least about 10%, at least about 15%, and at least about 20% solids. The aqueous shellac may have less than about 30% or less than about 25% solids. The aqueous shellac may be in solution at a range of about 5% to about 30% solids. The aqueous shellac may have about 20% to about 30% solids, particularly about 25% solids. Examples of commercially-available shellacs include, but are not limited to, MarCoat™ 125 (available from Emerson Resources) and Aqueous 125 (available from Parker Ingredients). MarCoat™ 125 contains dewaxed and decolorized shellac, methyl paraben, propyl paraben, isopropyl alcohol, and water. Aqueous 125 contains ammoniated shellac, denatured alcohol, potassium sorbate, and water. Ammoniated shellac is used embodiments of the invention. Commonly, an ammoniated shellac (as approved for food use) may exhibit different solubility characteristics in various pH, may be readily available and may be economical.

Traditionally, shellac coats have been sprayed from an organic solution, a disadvantage in terms of solution cost and environmental protection cost. It is possible to spray shellac from an aqueous solution after forming the shellac into a water soluble alkali salt, and aqueous shellac salt solutions are commercially available. These commercially available solutions form films that dissolve in neutral or mildly alkaline conditions and appear, at first consideration, to overcome the alkaline insolubility problem of shellac sprayed from organic solution. However, these films react rapidly in acid to revert to the free acid shellac and, when ingested as a film of a dosage unit, the acidic conditions in the stomach restore the film to shellac and restore the insolubility problem. Shellac films sprayed as shellac or as shellac salts perform similarly and neither resists acid (0.1 M HCl for two hours) and rapidly (within one hour) releases the contents of the dosage unit in neutral or mildly alkaline conditions in the manner of an enteric coat. Shellac films can be produced that disintegrate between two and three hours and would appear to meet the above requirements. However, shellac films are typically relatively insensitive to pH and, as described above, disintegrate between two and three hours regardless of the solution acidity or alkalinity and instead behave as erodible films which dissolve as a function of time.

Dewaxed orange shellac and refined bleached shellac may be used because of their low wax content. Orange shellac commonly has an acid value of about 68-71 and tends to form a better, glossier, and more impervious film. Bleached shellac commonly has an acid value of about 78-90 and tends to get tacky and forms a more permeable film. Both grades may be prepared as aqueous solutions using, for example, ammonium carbonate and/or ammonium hydroxide to solubilize them in water, and, if necessary a mixture of alcohol and water. Such aqueous solutions may comprise at least about 5%, at least about 10%, at least about 15%, or at least about 20% solids. The aqueous solutions may comprise less than about 30%, less than about 25%, less than about 24%, less than about 23%, less than about 22%, less than about 21%, less than about 20%, less than about 15%, or less than about 10% solids. This includes about 5% to about 30%, about 5% to about 25%, and about 10% to about 25% solids. The aqueous solutions may be prepared at temperatures of at least about 15°C, at least about 20°C, at least about 25 °C, at least about 30 °C, at least about 40 °C, at least about 50 °C, or at least about 60°C. The aqueous solutions may be prepared at temperatures of less than about 85°C, less than about 80°C, less than about 75°C, less than about 70°C, less than about 65°C, less than about 60°C, or less than about 50°C. This includes temperatures of about 15°C to about 85°C, and about 20°C to about 80°C.

Examples of polymers include, but not limited to, alginates which include, but are not limited to, sodium alginate, potassium alginate, and combinations thereof. Alginic acid, other salts of alginic acid (alginates), or alginic acid derivatives may also be used. Some examples of alginates may have a viscosity measured at 3% solids in water from about 20 to about 2500 mPa.s (about 20 to about 2500 centipoise), from about 50 to about 2,000 mPa.s (about 50 to about 2,000 centipoise), and particularly, from about 100 to about 1000 mPa.s (about 100 to about 1,000 centipoise), as well as a viscosity measured at 1% solids in water from about 5 to about 150 mPa.s (about 5 to about 150 centipoise), from about 10 to about 100 mPa.s (about 10 to about 100 centipoise), and particularly, from about 10 to about 40 mPa.s (about 10 to about 40 centipoise), and combinations thereof. In one embodiment, sodium alginate is used. Examples of commercially available sodium alginates include, but are not limited to, Protanal (available from FMC Corp.) and Manucol™ (available from ISP Technologies, Inc.). Protanal and Manucol™ comprise dried sodium alginate. When Protanal is mixed in water to a 3% solution, the resulting solution has a viscosity of about 100-1000 mPa.s (about 100-1,000 centipoise). When Manucol™ is mixed in water to a 1% solution, the resulting solution has a viscosity of about 10-40 mPa.s (about 10-40 centipoise). Sodium alginate is commercially available as different grades that form solutions of varying viscosities. Commonly, alginate, as approved for food use, may exhibit different solubility characteristics in various pH, may be readily available, and may be economical. In one embodiment, the water-miscible resin may comprise ammoniated shellac.

Examples of plasticizers include, but are not limited to, fatty acids, water-soluble plasticizers, water-insoluble plasticizers, triethyl citrate, triacetin, glycerin, propylene glycol, polypropylene glycol, polyethylene glycol (molecular weights of about 300 to about 8000), dibutyl sebacate, triglycerides, medium chain triglycerides (e.g., fractionated coconut oil), acetylated monoglycerides, glycerol monostearates, glycerin monostearate, oleic acid, polysorbates (such as polysorbate 80), stearic acid, sorbitol, tributyl citrate, acetyltributyl citrate, dibutyl phthalate, triethyl citrate, triethanolamine, and combinations thereof. A plasticizer may modify the flexibility of the film formed to suit dosage requirements. A plasticizer may enhance the film characteristics of the enteric coating, such as adhesion, flexibility, permeability, etc.

Examples of preservatives include, but are not limited to, benzalkonium chloride, benzoic acid, benzyl alcohol, benzoates, sorbates, nisin, natamycin, calcium priopionate, sorbic acid, sodium benzoate, methyl paraben, ethyl paraben, propyl paraben, butyl paraben, phenol, cresol, quaternary ammonium salts, potassium sorbate, and combinations thereof.

Examples of detackifying agents include, but are not limited to, aluminum hydrate, acetylated glycerides, diglycerides, acetylated monoglyceride, polyvinylpyrrolidone, sorbitan monostearate, polyglycerol esters, ethyl acetate, glyceryl monostearate, monoglycerides, poloxamers, polysorbates, stearic acid, sodium lauryl sulfate, triacetin, triethyl citrate, lecithins, mineral oil, talc, kaolin, and combinations thereof.

Examples of lubricants include, but are not limited to, talc, metallic stearates, silicon dioxide, sodium stearyl fumarate, palmitic acid, fatty acid esters, fatty acids, fatty alcohols, mineral oil, paraffins, leucine, polyethylene glycols, metallic lauryl sulfates, stearic acid, hydrogenated vegetable oil, and combinations thereof.

Examples of colorants include dyes, lakes, and pigments and may include, but are not limited to, titanium dioxide, iron oxides, dyes such as, for example, FD&C Lakes, Carmine Lake, FD&C Blue no. 1, FD&C Red no. 3, FD&C Red no. 40, FD&C Yellow no. 5, FD&C Yellow no. 6, FD&C Green no. 3, alumina, talc, annatto extract, calcium carbonate, canthaxanthin, caramel, β-carotene, carmine, dihydroxyacetone, tumeric oleoresin, cochineal extract, gardenia yellow, gardenia blue, beet powder, grape skin extract, riboflavin, chlorophyll-containing extracts, pearlescent pigments, SensiPearl™ Blue, Silver, and Bright Silver (available from Sensient Colors, Inc.), natural colorants, and the like. Other examples of colorants are found in 21 C.F.R. §§ 73 and 74, which are hereby fully incorporated by reference.

Examples of flavors may be synthetic or artificial flavors, natural flavors or any mixture thereof and may include, but are not limited to, flavenoids, antioxidants, natural flavorants, synthetic flavorants, bioflavenoids, flavones, flavone, flavonol, flavanonol, isoflavones, ethyl vanillin, tangerine flavor, lemon flavor, lemon extract, liquid caramel, spearmint oil, orange flavor, almond, amaretto, apple, green apple, apple-cherry-berry, apple-honey, apricot, bacon, balls of fire, banana, barbeque, beef, roast beef, beef steak, berry, berry blue, birch beer/spruce beer, blackberry, bloody mary, blueberry, boysenberry, brandy, bubble gum, butter, butter pecan, buttermilk, butterscotch, candy corn, cantaloupe, cantaloupe lime, caramel, carrot, cassia, caviar, celery, cereal, champagne, cherry, cherry cola, cherry maraschino, wild cherry, black cherry, red cherry, cherry-cola, chicken, chocolate, chocolate almond, cinnamon spice, citrus, citrus blend, citrus-strawberry, clam, cocoa, coconut, toasted coconut, coffee, coffee almond, cola, cola-vanilla, cookies & cream, cool, cotton candy, cranberry, cranberry-raspberry, cream, cream soda, dairy type cream, creme de menthe, cucumber, black currant, dulce de leche, egg nog, pork fat, type fat, anchovy fish, herring fish, sardine fish, frankfurter, fiery hot, fried garlic, sauteed garlic, gin, ginger ale, ginger beer, graham cracker type, grape, grape grapefruit, grapefruit-lemon, grapefruit-lime, grenadine, grill, guarana, guava, hazelnut, honey, hot, roasted honey, ice cream cone, jalapeno, key lime, kiwi, kiwi-banana, kiwi-lemon-lime, kiwi-strawberry, kola champagne, lard type, lemon, lemon custard, lemonade, pink lemonade, lemon-lime, lime, malt, malted milk, mango, mango-pineapple, maple, margarita, marshmallow, meat type, condensed milk, cooked milk, mint, mirepoix, mocha, mochacinna, molasses, mushroom, sauteed mushroom, muskmelon, nectarine, neopolitan, green onion, sauteed onion, orange, orange cordial, orange creamsicle, orange creme, orange peach mango, orange strawberry banana, creamy orange, mandarin orange, orange-passion-guava, orange-pineapple, papaya, passion fruit, peach, peachmango, peanut, roasted peanut, pear, pecan danish type, pecan praline, pepper, peppermint, pimento, pina colada, pina colada/pineapple-coconut, pineapple, pineapple-orange, pistachio, pizza, pomegranate, pork fat type, baked potato, prune, punch, citrus punch, tropical punch, cherry fruit punch, grape punch, raspberry, black raspberry, blue raspberry, red raspberry, raspberry-blackberry, raspberry-ginger ale, raspberry-lime, roast type, root beer, rum, sangria, sarsaparilla, sassafras, sausage, sausage pizza, savory, seafood, shrimp, hickory smoke, mesquite smoke, sour, sour cream, sour cream and onion, spearmint, spicy, strawberry, strawberry margarita, jam type strawberry, strawberry-kiwi, burnt sugar, sweet, supersweet, sweet & sour, tallow, tamarind, tangerine-lime, tangerine, tea, tequila type, toffee, triple sec, tropical fruit mix, turkey, tutti frutti, vanilla, vanilla cream, vanilla custard, french vanilla, vegetable, vermouth, vinegar, balsamic vinegar, watermelon, whiskey, wildberry, wine, and yogurt, and the like. Other examples of flavors are found in 21 C.F.R. §§172.510, 172.515, 172.520, 172.530, 172.535, 172.575, 172.580 and 172.585. A variety of food grade flavors are commercially available from Sensient Flavors Inc. in Indianapolis, Ind., Givaudan SA in Cincinnati, Ohio, and International Flavors & Fragrance in New York, N.Y.

Examples of sweeteners and/or taste maskants may include, but are not limited to, smoothenol, rosemary extract, aspartame, sucrose, honey, Magnasweet™, saccharin, sucralose, and the like.

Examples of emulsifiers include, but are not limited to, polysorbates (polyethoxylated sorbitan fatty acid derivatives) such as, for example, polysorbate 80; polyglyceryl 10 laurate; mono- and di-glycerides; propylene glycol; sodium lauryl sulfate; additives of propyl gallate and citric acid and stabilizers therein; alcohol; and combinations thereof.

Examples of buffering agents include, but are not limited to, sodium citrate.

Examples of antioxidants include, but are not limited to, tocopherol, rosemary extract, and combinations thereof.

Examples of solvents include, but are not limited to, ethanol, water, and combinations thereof.

The enteric coating composition of the invention may include (by weight) at least about 0.01%, at least about 0.02%, at least about 0.1 %, at least about 0.5%, at least about 1%, at least about 2%, at least about 3%, at least about 4%, at least about 5%, at least about 6%, at least about 7%, up to 8% of the resin. In some embodiments, the enteric coating composition may comprise less than 8%, less than 7%, less than 6%, less than 5%, less than 4%, less than 3%, less than 2%, or less than 1% resin. This includes, for example, 1% to 7% resin.

The enteric coating composition may comprise (by weight) at least about 0.01%, at least about 0.02%, at least about 0.1%, at least about 0.5%, at least about 1%, at least about 2%, at least about 3%, at least about 4%, at least about 5%, at least about 6%, at least about 7%, up to 8% of the polymer. In some embodiments, the enteric coating composition may comprise less than 8%, less than 7%, less than 6%, less than 5%, less than 4%, less than 3%, less than 2%, or less than 1% of the polymer. This includes, for example, 0.02% to 4% polymer.

In one embodiment, the enteric coating composition may contain equal quantities (by weight) of resin and polymer. In other embodiments, the enteric coating composition may contain about a 2:1 ratio, about a 3:1 ratio, about a 4:1 ratio, about a 5:1 ratio, about a 6:1 ratio, about a 7:1 ratio, about an 8:1 ratio, about a 9:1 ratio, and about a 10:1 ratio of resin to polymer.

The enteric coating composition may include (by weight) at least about 0%, at least about 1 %, at least about 2%, at least about 3%, at least about 4%, at least about 5%, at least about 6%, at least about 7%, at least about 8% plasticizer, at least about 9%, at least about 10%, at least about 15%, at least about 20%, and at least about 25% plasticizer. The enteric coating composition may include (by weight) less than about 35%, less than about 30%, less than about 25%, less than about 20%, and less than about 15% plasticizer. This includes, for example, about 0% to about 35%, from about 0% to about 30%, from about 0% to about 25%, and from about 1% to about 25% plasticizer. The plasticizer may comprise a food-approved, water-soluble, water-insoluble, or water-miscible plasticizer.

The enteric coating composition may include (by weight) at least about 0%, at least about 0.1 %, at least about 0.2%, at least about 0.3%, at least about 0.4%, at least about 0.5%, at least about 1%, at least about 2%, or at least about 3% preservative. The enteric coating composition may include (by weight) less than about 5%, less than about 4%, less than about 3%, less than about 2%, or less than about 1% preservative. This includes, for example, about 0% to about 5%, from about 0.1 % to about 5%, and from about 0.2% to about 4% preservative.

The enteric coating composition may include (by weight) at least about 0%, at least about 0.1 %, at least about 0.2%, at least about 0.3%, at least about 0.4%, at least about 0.5%, at least about 1%, at least about 5%, at least about 10%, or at least about 20% detackifying agent. The enteric coating composition may include (by weight) less than about 50%, less than about 45%, less than about 40%, less than about 35%, less than about 30%, less than about 25%, less than about 20%, and less than about 10% detackifying agent. This includes, for example, about 0% to about 50%, from about 0.1% to about 50%, and from about 0.5% to about 30% detackifying agent.

The enteric coating composition may include (by weight) at least about 0%, at least about 0.1 %, at least about 0.2%, at least about 0.3% lubricant, at least about 0.4%, at least about 0.5%, at least about 0.6%, at least about 0.7%, at least about 0.8%, at least about 0.9%, at least about 1%, at least about 2%, at least about 3%, at least about 4%, and at least about 5% lubricant. The enteric coating composition may include (by weight) less than about 10%, less than about 9%, less than about 8%, less than about 7%, less than about 6%, and less than about 5% lubricant. This includes, for example, about 0% to about 10% lubricant, about 0.2% to about 8%, and about 0.5% to about 7% lubricant. The lubricant may comprise food-approved, water-soluble, water-insoluble, or water-miscible lubricant.

The enteric coating composition may include (by weight) at least about 0%, at least about 0.01%, at least about 0.02%, at least about 0.03%, at least about 0.04%, at least about 0.05%, at least about 0.06%, at least about 0.07%, at least about 0.08%, at least about 0.09%, at least about 0.1 %, at least about 0.2%, at least about 0.3%, at least about 0.4%, at least about 0.5%, at least about 0.6%, at least about 0.7%, at least about 0.8%, at least about 0.9%, at least about 1%, at least about 1.2%, at least about 1.5%, and at least about 2% colorant. The enteric coating composition may include less than about 3%, less than about 2.5%, less than about 2%, less than about 1.5%, less than about 1.4%, less than about 1.3%, less than about 1.2%, less than about 1.1%, less than about 1%, and less than about 0.5% colorant. This includes, for example, from about 0.01% to about 3%, from about 0.06% to about 2%, and from about 0.1 % to about 1.2% colorant.

The enteric coating composition may include (by weight) at least about 0%, at least about 0.01%, at least about 0.02%, at least about 0.03%, at least about 0.04%, at least about 0.05%, at least about 0.06%, at least about 0.07%, at least about 0.08%, at least about 0.09%, at least about 0.1 %, at least about 0.2%, at least about 0.3%, at least about 0.4%, at least about 0.5%, at least about 0.6%, at least about 0.7%, at least about 0.8%, at least about 0.9%, at least about 1%, at least about 1.2%, and at least about 1.5% sweetener and/or taste maskant. The enteric coating composition may include less than about 2%, less than about 1.5%, less than about 1.4%, less than about 1.3%, less than about 1.2%, less than about 1.1 %, less than about 1%, less than about 0.9%, less than about 0.8%, less than about 0.7%, less than about 0.6%, and less than about 0.5% sweetener and/or taste maskant. This includes, for example, from about 0.01% to about 2% and from about 0.3% to about 1% sweetener and/or taste maskant.

The enteric coating composition may include (by weight) at least about 0%, at least about 0.01%, at least about 0.02%, at least about 0.03%, at least about 0.04%, at least about 0.05%, at least about 0.06%, at least about 0.07%, at least about 0.08%, at least about 0.09%, at least about 0.1 %, at least about 0.2%, at least about 0.3%, at least about 0.4%, at least about 0.5%, at least about 0.6%, at least about 0.7%, at least about 0.8%, at least about 0.9%, at least about 1%, at least about 1.2%, and at least about 1.5% flavor. The enteric coating composition may include less than about 2%, less than about 1.5%, less than about 1.4%, less than about 1.3%, less than about 1.2%, less than about 1.1%, less than about 1%, less than about 0.9%, less than about 0.8%, less than about 0.7%, less than about 0.6%, and less than about 0.5% flavor. This includes, for example, from about 0.01% to about 2% and from about 0.3% to about 1% flavor.

The enteric coating composition may include (by weight) at least about 0%, at least about 0.01%, at least about, at least about 0.02%, at least about 0.05%, at least about 0.1 %, at least about 0.2%, at least about 0.3%, at least about 0.4%, at least about 0.5%, at least about 0.6%, at least about 0.7%, at least about 0.8%, at least about 0.9%, at least about 1%, at least about 2%, and at least about 3% emulsifier. The enteric coating composition may include (by weight) less than about 5%, less than about 4%, less than about 3%, and less than about 2% emulsifier. This includes, for example, about 0% to about 5%, about 0.01% to about 5%, and about 0.1% to about 4% emulsifier. The emulsifier may comprise food-approved, water-soluble, water-insoluble, or water-miscible emulsifier.

The enteric coating composition may include (by weight) at least about 5%, at least about 10%, at least about 15%, at least about 20%, at least about 30%, at least about 40%, at least about 50%, at least about 60%, and at least about 70% solvent. The enteric coating composition may include (by weight) less than about 99%, less than about 98%, less than about 97%, less than about 96%, less than about 95%, less than about 94%, less than about 93%, less than about 92%, less than about 91%, less than about 90%, less than about 85%, less than about 80%, less than about 75%, and less than about 70% solvent. This includes, for example, about 5% to about 99%, about 25% to about 95%, and about 50% to about 90% solvent. The balance of the enteric coating composition may comprise solvent (e.g., water).

In some embodiments, the enteric coating composition may comprise a water-soluble shellac and a water-soluble alginate. At least one of the shellac and alginate may be less than 8%, less than 7%, less than 6%, less than 5%, less than 4%, less than 3%, less than 2%, or less than 1% (by weight) of the enteric coating composition. In one embodiment, bleached shellac (acid value 78-90, aqueous, 25% solids with ammonium hydroxide) is combined with sodium alginate at around a 2:1 ratio (solid shellac:sodium alginate) and also with plasticizers at 1-20% of resin. This may address the tackiness associated with bleached shellac.

In one embodiment, the disclosure provides an enteric coating composition that may include a de-waxed, de-colorized shellac ("flake shellac") and sodium alginate. As disclosed herein, but not presently claimed, the enteric coating composition may include at least one of an ammoniated shellac in an amount of about 24%, a sodium alginate in an amount of about 2.99%, glycerin in an amount of about 0.45%, acetylated monoglycerides in an amount of about 0.45%, polysorbate 80 in an amount of about 0.05%, stearic acid in an amount of about 0.01%, and water in an amount of about 72.05%.

The enteric coating composition may form an aqueous solution having a pH of at least about 5, at least about 6, and at least about 7. The enteric coating composition may form an aqueous solution having a pH of less than about 9, less than about 8, and less than about 7. This includes, for example, about 5 to about 9, 6 to 8, and about 7 to about 8. The pH of the enteric coating composition, or of components of the enteric coating composition, may be adjusted and selected to maintain a useable solution or suspension. The pH of the components before addition to the enteric coating composition may be at least about 5, at least about 6, at least about 6.5, at least about 6.6, at least about 6.7, at least about 6.8, at least about 6.9, and at least about 7. The pH of the components before addition to the enteric coating composition may be less than about 8.5, less than about 8, less than about 7.9, less than about 7.8, less than about 7.7, less than about 7.6, less than about 7.5, less than about 7.4, less than about 7.3, less than about 7.2, less than about 7.1, less than about 7, and less than about 6.9. This includes, for example, about 5 to about 8.5, about 6 to about 7, and about 6.8 to about 7.5. The enteric coating composition may be insoluble at low pH and soluble at a pH of greater than about 7, regardless of weight gain.

The enteric coating composition may have a viscosity of less than about 5,000, less than about 4,500, less than about 4,000, less than about 3,500, and less than about 3,000 mP.s (less than about 5,000, less than about 4,500, less than about 4,000, less than about 3,500, and less than about 3,000 centipoise). It may be in solution or in dispersion form.

In one embodiment, the enteric coating composition may be formed by adding water to a stainless steel container equipped with an overhead mixer. Into a vortex, the plasticizer may or may not be added. Into the vortex, using a dispersator or a high shear overhead mixer, polymer can be added and the mixture may be mixed for at least about 20 minutes, at least about 30 minutes, at least about 40 minutes, at least about 50 minutes, or at least 60 minutes to form a solution. Aqueous resin may then be added to the solution and the solution may be mixed for at least about 3 minutes, at least about 4 minutes, at least about 5 minutes, at least about 10 minutes, or at least about 20 minutes to form an enteric coating composition. The enteric coating composition can be used within about 12 hours, about 24 hours, or about 36 hours of preparation.

In one embodiment, the enteric coating composition may be formed from a multiple-component system (about 1% to about 12%, about 2% to about 11%, and about 5% to about 10%). The multiple-component system may comprise at least a first component comprising the ammoniated shellac and a second component comprising alginate. At least one of the first or second components may additionally comprise at least one of a plasticizer, a preservative, a detackifying agent, a lubricant, an emulsifier, a surfactant, a colorant, a flavor, a sweetener, a taste maskant, an opacifier, and combinations thereof in the first or second component. The components may be mixed to form an enteric coating composition. When the components or the enteric coating composition are applied to orally ingestible substrates (e.g., pharmaceutical tablets and dietary supplements), an enteric coating may be formed. An example of a multiple-component system includes a system in which a customer purchases a system comprising a separate first component comprising a shellac and a second component comprising an alginate, and then mixes the two components on site to form an enteric coating composition that is applied on site to form an enteric coating. In one example, without limitation, the first component may include at least one of a shellac and a plasticizer and the second component may include at least one of an alginate and a stearic acid.

In some embodiments, a subcoat may be applied to the substrate before coating with an enteric coating composition comprising about 0.01 % to about 10% resin and about 0.01 % to about 10% polymer. The subcoat may comprise, for example, a polymer, such as a cellulosic polymer, Kollidon™ VA 64, ethylcellulose, ChromaSeal™ ethylcellulose, and the like.

Coating can be done by applying an immediate release seal coat to the core, coating with an enteric coating composition including colorants and/or flavorants, and then an immediate top coat can also be applied to afford even more functionalities

The application techniques are described below. In some embodiments, a coating pan may be charged with capsules, tablets, and/or softgels. The bed may be warmed to at least about 18°C, at least about 19°C, at least about 20°C, at least about 21°C, at least about 22°C, at least about 23°C, at least about 24°C, at least about 25°C, at least about 26°C, at least about 27°C, at least about 28°C, at least about 29°C, and at least about 30°C. The bed may be warmed to less than about 42°C, less than about 41 °C, less than about 40°C, less than about 39°C, less than about 38°C, less than about 37°C, less than about 36°C, less than about 35°C, less than about 34°C, less than about 33°C, less than about 32°C, less than about 31 °C, less than about 30°C, and less than about 25°C. This includes, for example, about 18°C to about 42°C, about 20°C to about 40°C, and about 25°C to about 37°C. The processing parameters may be as set forth below.

The inlet temperature may be at least about 35°C, at least about 40°C, at least about 41°C, at least about 42°C, at least about 43°C, at least about 44°C, at least about 45°C, at least about 50°C, and at least about 55°C. The inlet temperature may be less than about 65°C, less than about 60°C, less than about 59°C, less than about 58°C, less than about 57°C, less than about 56°C, less than about 55°C, less than about 54°C, less than about 53°C, less than about 52°C, less than about 51 °C, and less than about 50°C. This includes inlet temperatures, for example, from about 35°C to about 65°C, about 40°C to about 65°C, and about 45°C to about 60 °C.

The outlet temperature may be at least about 20°C, at least about 25°C, at least about 28°C, at least about 30°C, at least about 31°C, at least about 32°C, at least about 33°C, at least about 34°C, at least about 35°C, at least about 36°C, at least about 37°C, at least about 38°C, at least about 39°C, at least about 40°C, at least about 41 °C, and at least about 42°C. The outlet temperature may be less than about 50°C, less than about 45°C, less than about 44°C, less than about 43°C, less than about 42°C, less than about 41 °C, and less than about 40°C. This includes, for example, outlet temperatures from about 20°C to about 50°C, from about 25 °C to about 45 °C, from about 28 °C to about 45 °C, from about 30 °C to about 45 °C, or from about 35 °C to about 42 °C.

The atomization pressure may be at least about 68.9, at least about 103, at least about 138, at least about 172, and at least about 207 kPa (at least about 10, at least about 15, at least about 20, at least about 25, and at least about 30 psi). The atomization pressure may be less than about 310, less than about 276, less than about 241, and less than about 207 psi (less than about 45, less than about 40, less than about 35, and less than about 30 psi). This includes, for example, atomization pressures from about 68.9 kPa (10 psi) to about 310 kPa (45 psi), from about 103 kPa (15 psi) to about 276 kPa (40 psi), and from about 207 kPa (30 psi) to about 276 kPa (40 psi). The air volume may be from about 23.6 l/s (50 cfm) to about 236 l/s (500 cfm).

The pan speed may be from about 3 rpm to about 22 rpm, about 8 rpm to about 20 rpm, about 11 rpm to about 19 rpm, and about 12 rpm to about 18 rpm. The spray rate may be from about 40 g/min to about 100 g/min, and about 50 g/min to about 80 g/min.

The solution solids may at least about 5%, at least about 6%, at least about 7%, at least about 8%, at least about 9%, at least about 10%, at least about 11%, at least about 12%, at least about 13%, at least about 14%, at least about 15%, at least about 20%, and at least about 25%. The solution solids may be less than about 35%, less than about 30%, less than about 20%, and less than about 15%.

The coating time may be from about 5 minutes to about 3 hours, about 10 minutes to about 2.5 hours, about 0.5 hours to about 2 hours, and about 1 hour to about 1.5 hours.

The enteric coating composition may be applied to a pharmaceutical or nutraceutical agent by loading at least about 5 Kg, at least about 10 Kg, at least about 11 Kg, at least about 12 Kg, at least about 13 Kg, at least about 14 Kg, or at least about 15 Kg of the pharmaceutical or nutraceutical agent into a vented coating pan, such as, for example, a 61 cm (24") side-vented coating pan (Compu-Lab 24). In some embodiments, the side-vented coating pan can be a 122 cm (48") pan, or a 152 cm (60") pan. The enteric coating composition can be applied under the following conditions. The inlet temperature may be about 40ºC to about 65ºC, the outlet temperature may be from about 20 °C to about 50 °C, from about 25 °C to about 45 °C, from about 30 °C to about 45 °C, or from about 35 °C to about 42 °C, the pan charge may be at least about 5 Kg, at least about 10 Kg, and at least about 15 Kg, the atomization pressure may be about 103 kPa (15 psi) to about 276 kPa (40 psi), the air volume may be about 23.6 l/s (50 cfm) to about 236 l/s (500 cfm), the pan speed may be about 3 rpm to about 11 rpm, the spray rate may be about 30 g/min to about 100 g/min, the solution solids may be about 10% to about 15%, the weight gain may be about 2.5% to about 4.5%, the coating efficiency may be about 75% to about 99%, and the coating time may be about 60 minutes to about 180 minutes. The enteric coating composition (in solution or suspension form) may, at a suitable concentration which is spraying system dependent, be sprayed using commercially available equipment to form films on dosage units.

The enteric coating composition may be in the form of a spray solution or a suspension, and may be applied to a pharmaceutical or nutraceutical agent using a vented coating pan (*i.e.,* sprayed). The enteric coating composition may be applied to coat substrates at a variety of coating temperatures (*e.g*., from about 28ºC to about 60ºC) and spray rates. The film may not be tacky and spray rate and temperature may not be factors. The solution can be allowed to dry, forming a dry enteric coating that can protect the pharmaceutical or nutraceutical agent from being attacked by acid of a stomach.

After drying, the enteric coating may include at least 1%, or at least 5%, up to 8% alginate.

After drying, the enteric coating may include at least 1%, or at least 5%, up to 8% of ammoniated shellac.

The enteric coatings may be a flexible film with tensile strength. The enteric coatings may possess one or more of the following characteristics: good adhesion to a substrate (such as, for example, cellulosic substrates or gelatin), smoothness of film, and clarity of film (transparent versus opaque). The enteric coating may be consistent batch-to-batch, may meet current FDA regulations for dietary supplements, may be robust (easy to use and prepare), and may coat various surfaces (gelatin, tablet, vegetable, etc). The enteric coating may meet USP/EP enteric standards for dietary supplements, nutraceuticals, and/or pharmaceuticals. The enteric coating may be cost effective and economical. Substrates coated with the enteric coatings may pass the USP31/NF26 enteric testing criteria for dietary supplements, Chapter 2040 (enteric-coated tablets - industry standard), and may pass the USP31/NF26 testing criteria at a variety of weight gains (about 3% to about 7%). The enteric coatings may pass the Disintegration Test, SGF TS, 1 hour (USP22 p. 1788) and may produce a result of less than 1 hour for the Disintegration Test, SIF TS, rupture time. (USP22 p. 1789). In some embodiments, the enteric coatings can be GRAS (Generally Recognized as Safe) and may be vegetarian. The enteric coatings may produce an amber translucent film on a softgel. The enteric coatings may be able to be applied at a fast rate and broad temperature while providing good film adhesion and flexibility.

Adhesion of the enteric coatings may be measured using a TA.TX Texture Analyzer (available from Texture Technologies Corp.) equipped with a 25 mm stainless steel cylindrical probe. A softgel coated with an enteric coating is scored around its hemisphere with a sharp blade and then attached to the top of the flat platform of the texture analyzer using heavy-duty double-sided tape (available from 3M). Another piece of heavy-duty double-sided tape is pressed to the bottom of the cylindrical probe, and the probe is then compressed to 800 g force onto the softgel for 10 seconds. The probe is then pulled away from the softgel at a rate of 1 mm/second, measuring the tension force until either (1) the coating separates from the softgel, or (2) the tape separates from the coating. Where the coating remains on the substrate, the measured force equals the force required to pull the double-sided tape away from the coating, and the adhesion force of the coating to the softgel is thus greater than the measured adhesion force between the tape and the coating. An adherent enteric coating may exhibit an adhesion force between the coating and the softgel of at least about 200 g force, at least about 400 g force, at least about 600 g force, at least about 700 g force, at least bout 800 g force, at least about 900 g force, and at least about 1000 g force.

Smoothness of the enteric coatings may be measured by utilizing a TA.TX Texture Analyzer with a vertical friction rig serving as a force arm. The probe height and force may be calibrated and the return force arm positioned 1 mm from the bottom of the container. A non-friction gliding object, such as a piece of paper tissue, may be attached to the vertical probe of the rig using heavy-duty double-sided tape. A tablet or other substrate coated with an enteric coating may be attached to the vertical wall of the rig using heavy-duty double-sided tape. A weight (e.g., 100 g) is added to the horizontal plate of the probe, and incremental increases of force are applied to the non-friction glidant in contact with the enteric coating until the glidant slips or moves to measure the tension force. A smooth enteric coating may exhibit a slip at a tension of at least about 10 g, at least about 20 g, and at least about 30 g tension force. A smooth enteric coating may exhibit a slip at a tension of less than about 100 g, less than about 90 g, less than about 80 g, less than about 70 g, less than about 60 g, less than about 50 g, less than about 40 g, less than about 30 g, and less than about 20 g tension force. A smooth enteric coating may exhibit a slip at a tension force from about 10 g to about 100 g, a tension force from about 20 g to about 70 g, and a tension force from about 30 g to about 50 g.

Clarity of the enteric coatings may be measured using a colorimeter (Labscan™ XE, available from Hunter Associates Laboratory, Inc.) to measure and compare the clarity between a coated and non-coated softgel. The coated and uncoated softgels are place on top of the orifice 0.318 cm (0.125 inch) of the colorimeter. Using a D65/10 measurement condition, the L, a, and b values can be obtained. The L value (Black/White indicated value) is used to indicate clarity, with a higher L value indicating greater opacity. Comparing the L values measured for the coated and uncoated softgels indicates the clarity of the coating. A softgel coated with a transparent enteric coating may exhibit an L value (D65/10) of at least about 4, at least about 5, at least about 6, at least about 7, at least about 8, at least about 9, at least about 10, at least about 15, and at least about 20. A softgel coated with a transparent enteric coating may exhibit an L value (D65/10) of less than about 50, less than about 40, less than about 30, less than about 20, less than about 15, less than about 10, less than about 9, less than about 8, and less than about 7. A softgel coated with a transparent enteric coating may exhibit an L value (D65/10) from about 4 to about 50, from about 6 to about 30, and from about 6 to about 25.

The enteric coating compositions may be used in food, pharmaceutical or nutraceutical applications intended for use in mammals, including, without limitation, rodents, canines, felines, non-human primates, ungulates, and humans. They may coat pharmaceutical or non-pharmaceutical dosage units. The enteric coating compositions may form an enteric coating that resists acid but disintegrates in neutral or mildly alkaline conditions. It may possess the properties of an enteric film, and have a controlled release profile such that it will release in an environment having a specified pH. The enteric coating may produce a controlled release profile in an environment having a selected pH based on a resin:polymer ratio.

### EXAMPLES

Exemplary embodiments of the present disclosure are provided in the following examples. The following examples are presented as illustrative of the disclosure and to assist one of ordinary skill in making and using the same. The examples are not intended in any way to otherwise limit the scope of the disclosure.
*Materials.* MarCoat™ 125 (available from Emerson Resources); Manucol™ LF (available from FMC BioPolymer); Myvacet™ acetylated monoglycerides (available from Kerry Bio-Science); Aqueous 125 (available from Parker Ingredients); ammoniated shellac; softgels, tablets, vitamin tablets, and capsules (available from Best Formulation); Kollidon™ VA (available from BASF); ChromaSeal™ (available from DuPont); Sucralose (available from JK Sucralose, Inc.); Magnasweet™ (available from MAFCO Worldwide Corp.); Smoothenol (available from Sensient Colors, Inc.); Herbalox™ seasoning type XT-P (rosemary extract) (available from Kalsec, Inc.); natural lemon extract, sweetened base with lemon flavor, and other flavors (available from Sensient Flavors, LLC); spearmint oil flavor (available from A.M. Todd Co.); Quick-Flo Honey Granules (available from Domino Specialty Ingredients).

### Method of Coating a Pharmaceutical or Nutraceutical Agent

An enteric coating composition may be applied to a pharmaceutical or nutraceutical agent using the following procedure. Load about 10 Kg to about 15 Kg of the pharmaceutical or nutraceutical agent into a vented coating pan, such as, for example, a 24" side-vented coating pan (Compu-Lab 242). A 122 cm (48") side-vented coating pan (Compu-Lab 48) may be suitable for a load of about 100 Kg to about 150 Kg. A 152 cm (60") side-vented coating pan (Compu-Lab 60) may be suitable for a load of about 300 Kg to about 350 Kg. The enteric coating composition can be applied to the pharmaceutical or nutraceutical agent under the following conditions. The inlet temperature may be about 40ºC to about 65ºC, the outlet temperature may be about 28ºC to about 45ºC, the atomization pressure may be about 103 kPa (15 psi) to about 276 kPa (40 psi), the air volume may be about 23.6 l/s (50 cfm) to about 236 l/s (500 cfm), the pan speed may be about 3 rpm to about 11 rpm, the spray rate may be about 30 g/min to about 100 g/min, the solution solids may be about 10% to about 15%, the weight gain may be about 2.5% to about 4.5%, the coating efficiency may be about 75% to about 99%, and the coating time may be about 60 minutes to about 180 minutes. These processing conditions are summarized in Table 1.

**Table 1. Enteric Coating Processing Conditions**

| **Processing Conditions** | **Target** | **Range** |
|---|---|---|
| Inlet (ºC) | 55 | 40-65 |
| Outlet (ºC) | 35 | 28-45 |
| Pan Charge (Kg) | 12 | 10-15 |
| Atomization Pressure (kPa) | 138 (20 psi) | 103-276 (15-40 psi) |
| Air Volume (l/s) | 165 (350 cfm) | 118-236 (250-500 cfm) |
| Pan Speed (rpm) | 7 | 3-11 |
| Spray rate (g/min) | 50 | 30-100 |
| Solution solids (%) | 10 | 10-15 |
| Weight gain (%) | 3.5 | 2.5-4.5 |
| Coating efficiency (%) | 85 | 75-99 |
| Coating time (min) | 90 | 60-120 |

### Example 1. Enteric Coating Composition

An enteric coating composition was prepared using ingredients as listed in Table 2.

**Table 2. Enteric Coating Ingredients**

| **Ingredient** | **% of Composition** |
|---|---|
| MarCoat™ 125 (25% solids) | 22.0% |
| Sodium alginate (Manucol™ LF) | 4.0% |
| Triethyl citrate | 1.4% |
| Water | 72.6% |
| | 100.0% |

The composition was prepared by using a dispersator or a high-shear over-head mixer to create a vortex in the water and then slowly adding the sodium alginate to the water vortex. The water and sodium alginate were mixed for at least 30 minutes to form an aqueous mixture. With continued mixing, the triethyl citrate and the MarCoat™ were added to the aqueous mixture. All ingredients were mixed for at least an additional 10 minutes to form an enteric coating composition. The formulation prepared with ingredients listed in Table 1 had 11% solids.

### Example 2. Enteric Coating Composition

An enteric coating composition was prepared using ingredients as listed in Table 3.

**Table 3. Enteric Coating Composition Ingredients**

| **Ingredient** | **% of Composition** |
|---|---|
| MarCoat™ 125 (25% solids) | 24.0% |
| Sodium alginate (Manucol™ LF) | 3.0% |
| Glycerin | 0.45% |
| Acetylated monoglycerides | 0.45% |
| Polysorbate 80 | 0.05% |
| Water | 72.05% |
| | 100.0% |

The composition was prepared by using a dispersator or a high-shear over-head mixer to create a vortex in the water and then slowly adding the sodium alginate to the water vortex. The water and sodium alginate were mixed for at least 30 minutes to form an aqueous mixture. With continued mixing, the glycerin, acetylated monoglycerides, polysorbate 80, and MarCoat™ were added to the aqueous mixture. All ingredients were mixed for at least an additional 10 minutes to form an enteric coating composition.

### Example 3. Enteric Coating Composition

An enteric coating composition was prepared using ingredients as listed in Table 4.

**Table 4. Enteric Coating Composition Ingredients**

| **Ingredients** | **% of Composition** |
|---|---|
| Aqueous 125 (25% solids) | 24.0% |
| Sodium alginate (Manucol™ LF) | 3.0% |
| Glycerin | 0.45% |
| Acetylated Monoglycerides | 0.45% |
| Polysorbate 80 | 0.05% |
| Stearic Acid | 0.05% |
| Water | 72.0% |
| | 100.0% |

The composition was prepared by using a dispersator or a high-shear over-head mixer to create a vortex in the water and then slowly adding the sodium alginate and stearic acid to the water vortex. The ingredients were mixed for at least 30 minutes to form an aqueous mixture. With continued mixing, the glycerin, acetylated monoglycerides, polysorbate 80, and Parker Ingredients were added to the aqueous mixture. All ingredients were mixed for at least an additional 10 minutes to form an enteric coating composition.

### Reference Example 4a. Enteric Coating Composition

An enteric coating composition was prepared using ingredients as listed in Table 5.

**Table 5. De-waxed De-colorized shellac ("Flake Shellac") Formulation**

| Ingredient | % |
|---|---|
| Ammoniated shellac | 24.00% |
| Sodium Alginate | 2.99% |
| Glycerin | 0.45% |
| Acetylated Monoglycerides | 0.45% |
| Polysorbate 80 | 0.05% |
| Stearic Acid | 0.01% |
| Water | 72.05% |
| Total Solution | 100.00% |

The composition was prepared by using a dispersator or a high-shear over-head mixer to create a vortex in the water and then slowly adding the sodium alginate and stearic acid to the water vortex. The ingredients were mixed for at least 30 minutes to form an aqueous mixture. With continued mixing, the glycerin, acetylated monoglycerides, polysorbate 80, and ammoniated shellac were added to the aqueous mixture. All ingredients were mixed for at least an additional 10 minutes to form an enteric coating composition.

### Example 4b. Composition

**Table 6. Composition Formulation**

| Ingredient | % |
|---|---|
| Ammoniated shellac | 25.00% |
| Glycerin | 0.45% |
| Acetylated Monoglycerides | 0.45% |
| Polysorbate 80 | 0.05% |
| Water | 74.05% |

The composition was prepared by using a dispersator or a high-shear over-head mixer to create a vortex in the water and then slowly adding the ammoniated shellac, glycerin, acetylated monoglycerides, and polysorbate 80 to the water vortex. The ingredients were mixed for at least 30 minutes to form an aqueous mixture.

### Example 4c. Composition

**Table 7. Composition Formulation**

| Ingredient | % |
|---|---|
| Sodium Alginate | 2.99% |
| Stearic Acid | 0.01% |
| Water | 97.00% |

The composition was prepared by using a dispersator or a high-shear over-head mixer to create a vortex in the water and then slowly adding the sodium alginate and stearic acid to the water vortex. The ingredients were mixed for at least 30 minutes to form an aqueous mixture.

### Example 5. Softgel Coating with Enteric Coating Composition

The composition according to Example 1 was used to coat Omega Smart (small oval softgels, ∼1.27 cm (∼ ½ inch)), Product JH0421, Lot J07033 (available from Best Formulations). The product was prepared and evaluated as follows.

**Table 6. Preparation and Testing of Coated Softgels**

| | |
|---|---|
| Pan Charge | 100 Kg |
| Solution solids | 10% |
| Spray rate | 120-150 g/min |
| Bed Temperature | 41ºC |
| Atomization Air Pressure | 5 bar |
| Coating Process | Good, minimal to no gun bearding |
| Coating finish | Average adhesion, good flexibility, spray-drying (white gelatin seams - poor coalescence) |
| USP Enteric test | Passed at 3 and 4% |

### Example 6. Softgel Coating with Enteric Coating Composition

The composition according to Example 1 was used to coat Omega Smart (small oval softgels, ∼1.27 cm (∼ ½ inch)), Product JH0421, Lot J07033 (available from Best Formulations). The product was prepared and evaluated as follows.

**Table 7. Preparation and Testing of Coated Softgels**

| | |
|---|---|
| Pan Charge | 100 Kg |
| Solution solids | 10% |
| Spray rate | 180 g/min |
| Bed Temperature | 36ºC |
| Atomization Air Pressure | 3.5 bar |
| Coating Process | Good, minimal to no gun bearding |
| Coating finish | Good adhesion, good flexibility, slight spray drying (very few white seams) |
| USP Enteric test | Passed at 3 and 4% |

**Table 7a. Preparation and Testing of Coated Softgels**

| | |
|---|---|
| Pan Charge | 100 Kg |
| Solution solids | 10% |
| Spray rate | 120-150 g/min |
| Bed Temperature | 41ºC |
| Atomization Air Pressure | 5 bar |
| Coating Process | Good, minimal to no gun bearding |
| Coating finish | Average adhesion, good flexibility, spray drying (white seams - poor coalescence) |
| USP Enteric test | Passed at 3 and 4% |

### Example 7. Softgel Coating with Enteric Coating Composition

The composition according to Example 2 was used to coat Omega Smart (small oval softgels, ∼1.27 cm (∼ ½ inch)), Product JH0421, Lot J07033 (available from Best Formulations). The product was prepared and evaluated as follows.

**Table 8. Preparation and Testing of Coated Softgels**

| System | Shellac-sodium alginate, two component system |
|---|---|
| Coating pan | 122 cm (48") |
| Coating preparation | good |
| | add ingredients to water, mix for 45 minutes |
| Pan charge | 100 Kg |
| Gun position | 25.4 cm (10") from coating surface |
| Solution solids | 10% |
| Spray rate (g/min) | 180 |
| Atomization Air Pressure (bar) | 3.5 |
| Solids (g/min) | 18 |
| Coating guns | 3 |
| Gun clogs/bearding | minimal/none |
| Agitate during application | No |
| Coating process | Good |
| Coating finish | good adhesion |
| | good flexibility |
| Cleanability | Moderate |
| USP enteric test | Pass |
| GRAS status | Yes |
| Vegetarian | Yes |

### Example 8. Multiple-Component Enteric Coating Composition for Dietary Supplements

The enteric coating composition was prepared by using a dispersator or a high-shear over-head mixer to create a vortex in 3.240 Kg of water and then slowly adding 0.135 Kg of the Example 4c composition to the water vortex. The ingredients were mixed for at least 30 minutes to form an aqueous mixture. With continued mixing, 1.125 Kg of the Example 4b composition was added to the aqueous mixture. All ingredients were mixed for at least an additional 10 minutes to form an enteric coating composition. The multiple-component enteric coating composition is summarized in Table 9.

**Table 9. Multiple-Component Enteric Coating Composition**

| Formulation: | | |
|---|---|---|
| | Weight Gain (%) | 3.0% |
| % of Composition | Total Solids (Kg) | 0.45 |
| 3.0% | Example 4c composition (Kg) | 0.135 |
| 25.0% | Example 4b composition (Kg) | 1.125 |
| 72.0% | Water (Kg) | 3.240 |
| 100.0% | Coating Solution (Kg) | 4.500 |

A 61 cm (24") coating pan was charged with 15 Kg of softgels (Omega Smart small oval softgels, ∼1.27 cm (∼ ½ inch); available from Best Formulations) and the bed was warmed to 37°C. A coating device was filled with the multiple-component enteric coating composition, which was applied to the softgels under the conditions described in Table 10.

**Table 10. Processing Parameters for Softgel Enteric Coating**

| Processing Parameters | |
|---|---|
| Inlet (°C) | 45-65 |
| Outlet (°C) | 35-42 |
| Atomization Pressure (kPa) | 207-276 (30-40 psi) |
| Air Volume (l/s) | 94.4-189 (200-400 cfm) |
| Pan Speed (rpm) | 12 - 18 |
| Spray rate (g/min) | 50-80 |
| Solution solids | 10.0% |
| Coating time (hours) | 1-1.5 |

**Comments**

| | |
|---|---|
| Disintegration Test, SGF TS, 1 hour | Pass |
| Disintegration Test, SIF TS, rupture time | less than 1 hour |
| Softgel appearance | Amber translucent film |
| Film adhesion | Excellent |
| Film Flexibility | Excellent |

### Comparative Example 1.

Best Acrycoat L30D (methacrylic acid copolymer available from Blanver) was used to coat Omega Pure 600 (large oblong softgels, ∼2.54 cm (∼ 1 inch)), Product SG2692, Lot C09042 (available from Best Formulations). The product was prepared and evaluated as follows.

**Table 11. Preparation and Testing of Coated Softgels**

| | |
|---|---|
| Pan Charge | 100 Kg |
| Solution solids | 15% |
| Spray rate | 120 g/min |
| Bed Temperature | 34ºC |
| Atomization Air Pressure | 5 bar |
| Coating Process | Average, gun clogs about once an hour |
| Coating finish | Good adhesion, poor flexibility |

### Comparative Example 2.

Methacrylic acid copolymer available from Blanver) was used to coat Omega Pure 600 (large oblong softgels, ∼2.54 cm (∼ 1 inch)), Product SG2692, Lot C09042, available from Best Formulations. The product was prepared and evaluated as follows.

**Table 12. Preparation and Testing of Coated Softgels**

| | |
|---|---|
| Pan Charge | 100 Kg |
| Solution solids | 15% |
| Spray rate | 120 g/min |
| Bed Temperature | 34ºC |
| Atomization Air Pressure | 5 bar |
| Coating Process | Average, gun clogs about once an hour |
| Coating finish | Good adhesion, poor flexibility |

### Comparative Example 3.

Aquarius EN SCM 19142 Clear (available from Aqualon) was used to coat Omega Smart (small oval softgels, ∼2.54 cm (∼1/2 inch)), Product JH0421, Lot J07033 (available from Best Formulations). The product was prepared and evaluated as follows.

**Table 13. Preparation and Testing of Coated Softgels**

| | |
|---|---|
| System | Shellac-based, one component, dry system |
| Coating pan | 122 cm (48") |
| Coating preparation | difficult |
| | heat water to 60 °C, difficult to disperse, at least one hour |
| Pan charge | 100 Kg |
| Gun position | 25.4 cm (10") from coating surface |
| Solution solids | 20% |
| Spray rate (g/min) | 150 |
| Atomization Air Pressure (bar) | 5 |
| Solids (g/min) | 30 |
| Coating quns | 3 |
| Gun clogs/bearding | minimal/none |
| Agitate during application | Yes |
| Coating process | Good |
| Coating finish | good adhesion |
| | good flexibility |
| Cleanability | Moderate |
| USP enteric test | Failed |
| GRAS status | Yes |
| Vegetarian | Yes |

### Comparative Example 4.

Nutrateric (available from Colorcon) was used to coat softgels available from Best Formulations. The product was prepared and evaluated as follows.

**Table 14. Preparation and Testing of Coated Softgels**

| | |
|---|---|
| System | Ethylcellulose-sodium alginate, two component system |
| Coating pan | 122 cm (48") |
| Coating preparation | Good |
| | add ingredients to water, mix for 60 minutes |
| Pan charge | 100 Kg |
| Gun position | 25.4 cm (10") from coating surface |
| Solution solids | 10% |
| Spray rate (g/min) | 150 |
| Atomization Air Pressure (bar) | 5 |
| Solids (g/min) | 15 |
| Coating guns | 3 |
| Gun clogs/bearding | Bearding |
| Agitate during application | Yes |
| Coating process | difficult, not robust, bed temperature critical |
| Coating finish | good adhesion |
| | good flexibility |
| Cleanability | Difficult |
| USP enteric test | Pass |
| GRAS status | Yes |
| Vegetarian | Yes |

### Comparative Example 5.

Acrycoat L30D (available from Blanver) was used to coat Omega Pure 600 (large oblong softgels, 2.54 cm (∼1 inch)), Product SG2692, Lot C09042 (available from Best Formulations). The product was prepared and evaluated as follows.

**Table 13. Preparation and Testing of Coated Softgels**

| | |
|---|---|
| Softgel coated | Omega Pure 600 (large oblong softgels, 2.54 cm (∼1inch), Product SG2692, Lot C09042, plus all other gelatin based softgels. Unable to coat veggie-softgels (too brittle) |
| System | Methylacrylic acid co-polymer, L100, no detackifier (talc or glycerol monostearate), plasticized with mineral oil |
| Coating pan | 122 cm (48") |
| Coating preparation | good |
| | add ingredients to water, mix for 30 minutes (cannot add ethyl vanillin) |
| Pan charge | 100Kg |
| Gun position | 25.4 cm (10") from coating surface |
| Solution solids | 15% |
| Spray rate (g/min) | 120 |
| Atomization Air Pressure (bar) | 5 |
| Solids (g/min) | 18 |
| Coating guns | 3 |
| Gun clogs/bearding | gun clog/stop to clean |
| Agitate during application | No |
| Coating process | difficult, very tacky - needs to be sprayed slowly |
| Coating finish | good adhesion |
| | poor flexibility (brittle) |
| Cleanability | Moderate |
| USP enteric test | Pass |
| GRAS status | No |
| Vegetarian | Yes |

### Example 9. Enteric Coating Compositions

**Table 14. Formulas for Enteric Coating Compositions**

| **Formulation** | | **Dissolution Testing** |
|---|---|---|
| Example 4b. | 23.00% | **Passed** |
| Example 4c. | 3.5% | Softgels and tablets from 8.09 to 6.0 in SGF and SIF. |
| Sorbitol 70% | 1.00% | |
| Water (g) | 72.50% | |
| Solution (g) | 100.00% | |
| | | |
| Example 4b. | 25% | **Passed** |
| Example 4c. | 3.00% | Acidic tablets coated to 2 and 2.7 wt % gains. Tablets were subcoated with 0.3% |
| Aspartame | 0.200% | |
| Ethyl Vanillin | 0.500% | |
| Titanium Dioxide | 6.000% | |
| Water (g) | 65.30% | ChromaSeal™ |
| Solution (g) | 100.00% | |
| Example 4b. | 25.00% | **Passed** |
| Example 4c. | 3.00% | Very Acidic and basic cores to 3% wt. gain. |
| Saccharin | 0.200% | |
| Ethyl Vanillin | 0.500% | **Passed** |
| Titanium Dioxide | 4.200% | A vitamin tablet (Citracal petite) at pH=6.1 |
| Talc | 1.800% | |
| Water (g) | 65.30% | |
| Solution (g) | 100.00% | |
| | | |
| Example 4b. | 25.0% | **Passed** |
| Example 4c. | 3.0% | Neutral tablets at 6.0 to 6.97 pH |
| Water (g) | 72.0% | |
| Solution (g) | 100.0% | |
| | | |
| Example 4b. | 25.00% | **Passed** |
| Example 4c. | 1.50% | Basic tablets Optimized range of Example 4c to Kollidon™ (1.5:1.5) |
| Kollidon™ VA 64 | 1.500% | |
| Water (g) | 72.00% | |
| Solution (g) | 100.00% | |
| | | |
| Example 4b. | 25.00% | **Passed** |
| Example 4c. | 0.80% | Basic tablets. This is the lowest range of Example 4c to Kollidon™ (0.8:0.8) |
| Kollidon™ VA 64 | 0.80% | |
| Water (g) | 73.40% | |
| Solution (g) | 100.00% | |
| | | |
| Example 4b. | 25.00% | **Passed** |
| Example 4c. | 3.00% | SIF when a 16.9% solution solid was made. |
| Aspartame | 0.200% | |
| Ethyl Vanillin | 0.500% | |
| Titanium Dioxide | 6.000% | |
| Water (g) | 65.30% | Core subcoated with |
| Solution (g) | 100.00% | 0.3% ChromaSeal™ |

### Example 10. Enteric Coating on 2-Piece Capsules

An enteric coating composition comprising 1% Example 4c composition and 5% Example 4b composition was coated onto the 2-piece capsules with the following parameters, and samples were withdrawn when weight gain reached 5, 7.5, 10 and 12%:

| Coating Parameters | |
|---|---|
| Inlet Temperature | 45 °C |
| Outlet Temperature | 38 °C |
| Pan Speed | 15 rpm |
| Pump Speed | 5-10 rpm |
| Air Flow | 27 l/s (57 cfm) |

All samples were tested in SGF and SIF disintegration. The results obtained are as follows:

| | SGF (min) | SIF (min) |
|---|---|---|
| 5% | >75 | 30 |
| 7.5% | >75 | 37 |
| 10% | >75 | 45 |
| 12% | >75 | 50 |

The composition including 1% Example 4c composition and 5% Example 4b composition with 5% weight gain passed SGF and SIF disintegration testing, and the SIF disintegration time was a direct function of weight gain percentage. See Figure 1. The coating was thin and elastic, with a slight amber color as compared to the uncoated substrate capsules.

### Example 11. Enteric Coating Composition - Flavored GRAS Enteric Tablets

| | | |
|---|---|---|
| **Example 4b** | 25 | 50.0g |
| **Example 4c** | 3 | 6.000g |
| **Aspartame** | 0.55 | 1.100g |
| **Ethyl Vanillin** | 0.25 | 0.500g |
| **Citric Acid** | 0.1 | 0.200g |
| **Water (g)** | 71.1 | 142.2g |
| **Solution (g)** | 100.00 | 200.0 |
| | **Solution solids %** | 10.9% |

### Example 12. Enteric Coating Composition - Flavored GRAS Enteric Tablets

| | | |
|---|---|---|
| **Example 4b** | 25.00 | 50.0g |
| **Example 4c** | 3.00 | 6.000g |
| **Aspartame** | 0.20 | 0.400g |
| **Tangerine Flavor** | 0.80 | 1.600g |
| **Water (g)** | 71.00 | 142.0g |
| **Solution (g)** | 100.00 | 200.0 |
| | **Solution solids %** | 11.0% |

### Example 13. Enteric Coating Composition - Flavored GRAS Enteric Tablets

| | | |
|---|---|---|
| **Example 4b** | 25.00 | 50g |
| **Example 4c** | 3.00 | 6.000g |
| **Aspartame** | 0.25 | 0.500g |
| **Sweetened base with Lemon Flavor** | 0.05 | 0.100g |
| **Water (g)** | 71.70 | 143.4g |
| **Solution (g)** | 100 | 200.0 |
| | **Solution solids %** | 10.3% |

### Example 14. Enteric Coating Composition - Flavored GRAS Enteric Tablets

| | | |
|---|---|---|
| **Example 4b** | 25 | 50g |
| **Example 4c** | 3 | 6.000g |
| **Aspartame** | 0.45 | 0.900g |
| **83% OH Lemon Extract** | 0.15 | 1.765g |
| **Water (g)** | 71.4 | 141.335g |
| **Solution (g)** | 100.00 | 200.0 |
| | **Solution solids %** | 10.6% |

### Example 15. Enteric Coating Composition - Flavored GRAS Enteric Tablets

| | | |
|---|---|---|
| **Example 4b** | 25.00 | 50g |
| **Example 4c** | 3.00 | 6.000g |
| **Aspartame** | 0.60 | 1.200g |
| **Sweetened Base with Lemon Flavor** | 2.00 | 4.000g |
| **Water (g)** | 69.40 | 138.8g |
| **Solution (g)** | 100.00 | 200.0 |
| | **Solution solids %** | 12.6% |

### Example 16. Enteric Coating Composition - Flavored GRAS Enteric Tablets

| | | |
|---|---|---|
| **Example 4b** | 25.0 | 50g |
| **Example 4c** | 3.0 | 6.000g |
| **Sweetened Base with Lemon Flavor** | 4.0 | 8.000g |
| **Water (g)** | 68.0 | 136g |
| **Solution (g)** | 100 | 200.0 |
| | **Solution solids %** | 14.0% |

### Example 17. Enteric Coating Composition - Flavored GRAS Enteric Tablets

| | | |
|---|---|---|
| **Example 4b** | 25.00 | 50.00g |
| **Example 4c** | 3.00 | 6.00g |
| **Aspartame** | 0.50 | 1.00g |
| **Sweetened Base with Lemon Flavor** | 2.00 | 4.00g |
| **Water (g)** | 69.50 | 139.00g |
| **Solution (g)** | 100 | 200.0 |
| | **Solution solids %** | 12.5% |

### Example 18. Enteric Coating Composition - Lemon Flavored GRAS Enteric Tablets

| | | |
|---|---|---|
| **Example 4b** | 25 | 50g |
| **Example 4c** | 3 | 6.000g |
| **Aspartame** | 0.5 | 1.000g |
| **Sweetened Base with Lemon Flavor** | 1.5 | 3.000g |
| **Water (g)** | 70 | 140g |
| **Solution (g)** | 100 | 200.0 |
| | **Solution solids %** | 12.0% |

### Example 19. Enteric Coating Composition - Lemon Flavored GRAS Enteric Tablets

| | | |
|---|---|---|
| **Example 4b** | 25.00 | 50.00g |
| **Example 4c** | 3.00 | 6.000g |
| **Spearmint Oil Flavor** | 0.12 | 2.400g |
| **Water (g)** | 71.88 | 141.60g |
| **Solution (g)** | 100.00 | 200.0 |
| | **Solution solids %** | 10.1% |

### Example 20. Enteric Coating Composition - Flavored GRAS Enteric Softgels

| | | |
|---|---|---|
| **Example 4b** | 24.380 | 48.760g |
| **Example 4c** | 2.930 | 5.860g |
| **Sucralose** | 0.250 | 0.500g |
| **Riboflavin** | 0.400 | 0.800g |
| **SensiPearl™ Silver** | 0.240 | 0.480g |
| **Titanium Dioxide** | 1.480 | 2.960g |
| **Ethyl Vanillin** | 0.150 | 0.300g |
| **Water (g)** | 70.170 | 140.34g |
| **Solution (g)** | 100.00 | 200.00 |
| | **Solution solids %** | 12.276% |

### Example 21. Enteric Coating Composition - Flavored GRAS Enteric Tablets

| | | |
|---|---|---|
| **Example 4b** | 15.00 | 30.00g |
| **Example 4c** | 4.00 | 8.000g |
| **Carmine Lake** | 0.50 | 1.000g |
| **SensiPearl™ Bright Silver** | 8.80 | 17.600g |
| **Water (g)** | 71.70 | 143.4g |
| **Solution (g)** | 100.00 | 200.0 |
| | **Solution solids %** | 17.5% |

### Example 22. Enteric Coating Composition - Iridized GRAS Enteric Tablets

| | | |
|---|---|---|
| **Example 4b** | 25.00 | 50.00 |
| **Example 4c** | 3.00 | 6.00 |
| **Carmine Lake** | 0.50 | 1.00 |
| **SensiPearl™ Bright Silver** | 4.50 | 9.00 |
| **Water (g)** | 67.00 | 134.00 |
| **Solution (g)** | 100.00 | 200.00 |
| | **Solution solids %** | 15.0% |

### Example 23. Enteric Coating Composition - Iridized GRAS Enteric Tablets

| | | |
|---|---|---|
| **Example 4b** | 25 | 50g |
| **Example 4c** | 3 | 6.000g |
| **Carmine Lake** | 0.5 | 1.000g |
| **SensiPearl™ Bright Silver** | 2 | 4.000g |
| **Water (g)** | 69.5 | 139.0g |
| **Solution (g)** | 100.0 | 200.0 |
| | **Solution solids %** | 12.5% |

### Example 24. Enteric Coating Composition - Iridized GRAS Enteric Tablets

| | | |
|---|---|---|
| **Example 4b** | 25 | 50g |
| **Example 4c** | 3 | 6.000g |
| **Carmine Lake** | 0.1 | 0.200g |
| **SensiPearl™ Bright Silver** | 0.5 | 1.000g |
| **Water (g)** | 71.4 | 142.8g |
| **Solution (g)** | 100.0 | 200.0 |
| | **Solution solids %** | 10.6% |

### Example 25. Enteric Coating Composition - Pearlescent Brown GRAS Enteric Tablets

| | | |
|---|---|---|
| **Example 4b** | 25 | 50g |
| **Example 4c** | 3 | 6.000g |
| **Liquid Caramel** | 0.6 | 1.200g |
| **SensiPearl™ Bright Silver** | 0.5 | 1.000g |
| **Water (g)** | 70.9 | 141.8g |
| **Solution (g)** | 100.0 | 200.0 |
| **Solution solids %** | | 11.1% |
| **Specific Gravity** | | 0.993 |
| **pH** | | 7.300 |
| **Sp. 63, 100 rpm, 72%, 19C - Viscosity (cps)** | | 872.0 |

### Example 26. Enteric Coating Composition - GRAS Brown Enteric Tablets

| | | |
|---|---|---|
| **Example 4b** | 25 | 50g |
| **Example 4c** | 3 | 6.0g |
| **Liquid Caramel** | 0.6 | 1.2g |
| **Water (g)** | 71.4 | 142.8g |
| **Solution (g)** | 100.0% | 200.0g |
| **Solution solids %** | | 10.6% |
| **Specific Gravity** | | 1.016 |
| **pH** | | 7.400 |
| **Sp. 63, 100 rpm, 59.8%, 19C - Viscosity (cps)** | | 717.4 |

### Example 27. Enteric Coating Composition - GRAS Red Enteric Tablets

| | | |
|---|---|---|
| **Example 4b** | 25 | 50g |
| **Example 4c** | 3 | 6.000g |
| **Carmine Lake** | 0.15 | 0.300g |
| **Water (g)** | 71.85 | 143.7g |
| **Solution (g)** | 100.0% | 200.0g |
| **Solution solids %** | | 10.15% |
| **Specific Gravity** | | 1.021 |
| **pH** | | 7.500 |
| **Sp. 62, 100 rpm, 72.4%, 19C - Viscosity (cps)** | | 868.6 |

### Example 28. Enteric Coating Composition - Lemon Flavored GRAS Enteric Tablets

| | | |
|---|---|---|
| **Example 4b** | 25 | 50g |
| **Example 4c** | 3 | 6.000g |
| **Magnasweet™** | 0.5 | 1.000g |
| **Lemon Flavor** | 0.5 | 1.000g |
| **Water (g)** | 71.0 | 142.0g |
| **Solution (g)** | 100.0% | 200.0g |
| **Solution solids %** | | 11.0% |
| **Specific Gravity** | | 1.000 |
| **pH** | | 7.000 |
| **Sp. 62, 12 rpm, 40%, 25C - Viscosity (cps)** | | 825 |

### Example 29. Enteric Coating Composition - Lemon Flavored GRAS Enteric Tablets

| | | |
|---|---|---|
| **Example 4b** | 25 | 50g |
| **Example 4c** | 3 | 6.000g |
| **Smoothenol** | 0.2 | 0.400g |
| **Lemon Flavor** | 0.2 | 0.400g |
| **Water (g)** | 71.6 | 143.2g |
| **Solution (g)** | 100.0% | 200.0g |
| **Solution solids %** | | 10.4% |

### Example 30. Enteric Coating Composition - Orange Mandarin Flavored GRAS Enteric Tablets

| | | |
|---|---|---|
| **Example 4b** | 25 | 50g |
| **Example 4c** | 3 | 6.000g |
| **Herbalox™ (Rosemary Extract)** | 0.25 | 0.500g |
| **Orange Mandarin Flavor** | 0.5 | 1.000g |
| **Water (g)** | 71.25 | 142.5g |
| **Solution (g)** | 100.0% | 200.0g |
| **Solution solids %** | | 10.75% |
| **Specific Gravity** | | 0.996 |
| **pH** | | 7.300 |
| **Sp. 62, 12 rpm, 40%, 25C - Viscosity (cps)** | | 688 |

### Example 31. Enteric Coating Composition - Orange Mandarin Flavored GRAS Enteric Softgels

| | | |
|---|---|---|
| **Example 4b** | 25 | 50g |
| **Example 4c** | 3 | 6.000g |
| **Herbalox™ (Rosemary Extract)** | 0.03 | 0.060g |
| **Tocopherol** | 0.03 | 0.060g |
| **Lemon Flavor** | 0.3 | 0.600g |
| **Water (g)** | 71.64 | 143.3g |
| **Solution (g)** | 100.0% | 200.0g |
| **Solution solids %** | | 10.36% |

### Example 32. Enteric Coating Composition - Orange Mandarin Flavored GRAS Enteric Tablets

| | | |
|---|---|---|
| **Example 4b** | 25 | 50g |
| **Example 4c** | 3 | 6.000g |
| **Magnasweet™** | 0.05 | 0.100g |
| **Honey granules** | 0.3 | 0.600g |
| **Orange Mandarin Flavor** | 0.3 | 0.600g |
| **Water (g)** | 71.35 | 142.7g |
| **Solution (g)** | 100.0% | 200.0g |
| **Solution solids %** | | 10.65% |

### Example 33. Enteric Coating Composition - Lemon Flavored Enteric Coated Softgels

| | | |
|---|---|---|
| **Example 4b** | 25 | 50g |
| **Example 4c** | 3 | 6.000g |
| **Magnasweet™** | 0.2 | 0.400g |
| **Sucralose** | 0.1 | 0.200g |
| **Lemon Flavor** | 1.5 | 3.000g |
| **Herbalox™ (Rosemary Extract)** | 0.1 | 0.200g |
| **Water (g)** | 70.1 | 140.2g |
| **Solution (g)** | 100 | 200.0 |
| **Solution solids %** | | 11.90% |
| **Specific Gravity (g/mL)** | | 0.936 |
| **pH** | | 7.067 |
| **Sp. 63, 100RPM , 58.9 %, 25C Viscosity cps** | | 707 |

### Example 34. Enteric Coating Composition - Orange Flavored Enteric Coated Softgels

| | | |
|---|---|---|
| **Example 4b** | 25 | 50g |
| **Example 4c** | 3 | 6.000g |
| **Magnasweet™** | 0.2 | 0.400g |
| **Sucralose** | 0.1 | 0.200g |
| **Orange Flavor** | 1.5 | 3.000g |
| **Herbalox™ (Rosemary Extract)** | 0.1 | 0.200g |
| **Water (g)** | 70.1 | 140.2g |
| **Solution (g)** | 100 | 200.0 |
| **Solution solids %** | | 11.90% |
| **Specific Gravity (g/mL)** | | 0.935 |
| **pH** | | 7.091 |
| **Sp. 63, 100RPM , 58 %, 25C Viscosity cps** | | 696 |

### Example 35. Enteric Coating Composition - Pearlescent Blue Enteric Tablets

| | | |
|---|---|---|
| **Example 4b** | 25 | 50g |
| **Example 4c** | 3 | 6.000g |
| **FD&C Al Lake Blue No.1** | 0.025 | 0.050g |
| **SensiPearl™ Blue** | 0.5 | 1.000g |
| **Water (g)** | 71.5 | 143.0g |
| **Solution (g)** | 100.0 | 200.0 |
| | **Solution solids %** | 10.5% |
| **Specific Gravity** | | 1.020 |
| **pH** | | 7.090 |
| **Sp. 63, 100 RPM , 72 %, 19C Viscosity cps** | | 707.0 |

### Example 36. Enteric Coating Composition - Pearlescent Blue Enteric Tablets

| | | |
|---|---|---|
| **Example 4b** | 25 | 50g |
| **Example 4c** | 3 | 6.000g |
| **FD&C Blue No. 1 Dye** | 0.05 | 0.100g |
| **SensiPearl™ Blue** | 0.5 | 1.000g |
| **Water (g)** | 71.4 | 142.8g |
| **Solution (g)** | 100.0 | 200.0 |
| | **Solution solids %** | 10.55% |
| **Specific Gravity** | | 0.993 |
| **pH** | | 7.400 |
| **Sp. 62, 20 RPM , 40 %, 19C Viscosity cps** | | 610.0 |

### Example 37. Enteric Coating Composition - Pearlescent Red Coating

| | | |
|---|---|---|
| **Example 4b** | 25 | 50g |
| **Example 4c** | 3.0 | 6g |
| **Carmine Lake** | 0.1 | 0.2g |
| **SensiPearl™ Bright Silver** | 0.5 | 1g |
| **Water (g)** | 71.4 | 142.8g |
| **Solution (g)** | 100.0 | 200.0 |
| | **Solution solids %** | 10.6% |

### Example 38. Enteric Coating Composition - White GRAS Enteric Tablets

The substrate used was Nutra tablets coated with 0.3% Nutra Seal subcoat.

| | | |
|---|---|---|
| **Example 4b** | 25 | 150g |
| **Example 4c** | 3 | 18.000g |
| **Aspartame** | 0.2 | 1.200g |
| **Ethyl Vanillin** | 0.5 | 3.000g |
| **Titanium Dioxide** | 6 | 36.000g |
| **Water (g)** | 65.3 | 393 |
| **Solution (g)** | 100.0 | 600 |
| | **Solution solids %** | 16.9% |
| **Specific Gravity** | | 1.078 |
| **pH** | | 7.370 |
| **Sp. 63, 60 RPM, 67%, 25C Viscosity cps** | | 1348 |
| **Theoretical Solution for 2.7% Weight Gain** | | 118.3g |
| **Actual Solution Applied** | | 118.3g |
| **Finished Tablet Weight (kg)** | | 1.02 |

| | |
|---|---|
| Inlet (°C) | 50 |
| Outlet (°C) | 36-40 |
| Tablets Pan Charge (kg) | 1 |
| Atomization Pressure (kPa) | 152 (22 psi) |
| Air Volume (l/s) | 118 (250 cfm) |
| Pan Speed | 12 |
| Average Spray Rate (g/min) | 14.8 |
| Solution Solids | 16.9% |
| Actual Weight Gain | 2.0% |
| Coating Accountability | 100.0% |
| Coating Time (min) | 8 |

| | **Unwaxed** | **TOP Coat SB White 63-003** |
|---|---|---|
| Nutra Tablets (shellac coated pan) at 36°C | 1.43% | 2.4% |
| Disintegration Test (SGF TS, 1 hour) | Pass | Pass |
| Disintegration Test, (SIF TS) Rupture Time | Two dissolved in 9 minutes | Passed (33 min) |

| | **Unwaxed** | **TOP Coat SB White 63-003** |
|---|---|---|
| Nutra Tablets (shellac coated pan) at 40 °C | 2.2% | 1.8% |
| Disintegration Test (SGF TS, 1 hour) | Passed | Passed |
| Disintegration Test, (SIF TS) Rupture Time | Passed | Passed (36 min) |
| | all three tablets were mushy throughout a semi intact film | |

### Example 39. GRAS Enteric Tablets with subcoat using a 10% SS of Kollidon™ VA 64

Substrates tested included calcium tablets (pH 8.09, 0.98g); Creatine tablets (pH 2.24, 1.76g), gel capsules filled with fish oil. Kollidon™ VA 64 subcoat tested at 1.6% (23.5g) and 2.6% (33.1g).

| | | |
|---|---|---|
| **Example 4b** | 25 | 50g |
| **Example 4c** | 3 | 6.0g |
| **Water (g)** | 72 | 144.0g |
| **Solution (g)** | 100.0% | 200.0g |
| **Theoretical Solution for 2.7% Weight Gain** | | 40.0g |
| **Actual Solution Applied** | | 34g |
| **Finished Tablet Weight (kg)** | | 0.154 |

| | |
|---|---|
| Inlet (°C) | 50 |
| Outlet (°C) | 39.9 |
| Tablets Pan Charge (kg) | 0.15 |
| Atomization Pressure (kPa) | 152 (22 psi) |
| Air Volume (l/s) | 27 (57 cfm) |
| Pan Speed | 12 |
| Average Spray Rate (g/min) | 6.0 |
| Solution Solids | 10.2% |
| Actual Weight Gain | 2.7% |
| Coating Accountability | 117.6% |
| Coating Time (min) | 8 |

| | | |
|---|---|---|
| **Calcium Tablet** | **1.6%** | **2.6%** |
| Disintegration Test (SGF TS) 1 hour | Passed | Passed |
| Disintegration Test (SIF TS) Rupture Time | Passed 30 min | Passed |
| Film Adhesion (scraped with ss spatula) | completely gone | |
| **Creatine Tablet** | **1.6%** | **2.6%** |
| Disintegration Test (SGF TS) 1 hour | Passed | Passed |
| Disintegration Test (SIF TS) Rupture Time | 2 dissolved, 1 didn't | 2 mushy all the way through, 1 completely dissolved |
| **Fish Oil gel capsules** | **1.6%** | **2.6%** |
| Disintegration Test (SGF TS) 1 hour | Failed | Passed |
| Disintegration Test (SIF TS) Rupture Time | | Passed |

### Example 40. GRAS Enteric Tablets with subcoat using a 10% SS of Kollidon™ VA 64

Substrates tested included calcium tablets (pH 8.09, 0.98g); Nutra tablets (pH 2.24, 1.76g); placebo tablets (pH 6.97, 0.31g); gel capsules filled with fish oil.

| | | |
|---|---|---|
| **Example 4b** | 25 | 50g |
| **Example 4c** | 3 | 6.0g |
| **Water (g)** | 72 | 144.0g |
| **Solution (g)** | 100.0% | 200.0g |
| **Specific Gravity** | | 1.024 |
| **pH Solution** | | 7.240 |
| **Viscosity (s63, 20rpm, 25 °C, 72.2%) (cps)** | | 900 |
| **Theoretical Solution for 2.7% Weight Gain** | | 35 |
| **Actual Solution Applied** | | 32 |
| **Finished Tablet Weight (kg)** | | 0.154 |
| Inlet (°C) | 50 | |
| Outlet (°C) | 39.9 | |
| Tablets Pan Charge (kg) | 0.15 | |
| Atomization Pressure (kPa) | 152 (22 psi) | |
| Air Volume (l/s) | 27 (57 cfm) | |
| Pan Speed | 12 | |
| Average Spray Rate (g/min) | 6.0 | |
| Solution Solids | 10.2% | |
| Actual Weight Gain | 2.7% | |
| Coating Accountability | 109.4% | |
| Coating Time (min) | 12 | |

| **Calcium Tablets** | **1.7%** | **2.7%** |
|---|---|---|
| Disintegration Test (SGF TS) 1 hour | Failed | Passed |
| Disintegration Test (SIF TS) Rupture Time | | Passed |

| **Nutra Tablets** | **1.7%** | **2.7%** |
|---|---|---|
| Disintegration Test (SGF TS) 1 hour | Passed | Passed |
| Disintegration Test (SIF TS) Rupture Time | Passed, 20 min | Passed, 40 min |

| **Placebo Tablets** | **1.7%** | **2.7%** |
|---|---|---|
| Disintegration Test (SGF TS) 1 hour | failed, 30 min | n/a |

| **Fish Oil gel capsules** | **1.6%** | **2.6%** |
|---|---|---|
| Disintegration Test (SGF TS) 1 hour | Failed | Passed |
| Disintegration Test (SIF TS) Rupture Time | | Passed |

### Example 41. GRAS Enteric Tablets with subcoat using a 10% SS of Kollidon™ VA 64

Substrates tested included calcium tablets (pH 8.09, 0.98g); creatine tablets (pH 2.24, 1.76g); placebo tablets (pH 6.97, 0.31g).

| | | |
|---|---|---|
| **Example 4b** | 25 | 50g |
| **Example 4c** | 3 | 6.0g |
| **Water (g)** | 72 | 144.0g |
| **Solution (g)** | 100.0% | 200.0g |
| **Specific Gravity** | 1.024 | |
| **pH Solution** | 7.240 | |
| **Viscosity (s63, 20rpm, 25°C, 72.2%) (cps)** | 900 | |
| **Theoretical Solution for 2.7% Weight Gain** | 40g | |
| **Actual Solution Applied** | 34g | |
| **Finished Tablet Weight (kg)** | 0.154 | |

| | |
|---|---|
| Inlet (°C) | 50 |
| Outlet (°C) | 39.9 |
| Tablets Pan Charge (kg) | 0.15 |
| Atomization Pressure (kPa) | 152 (22 psi) |
| Air Volume (l/s) | 27 (57 cfm) |
| Pan Speed | 12 |
| Average Spray Rate (g/min) | 6.0 |
| Solution Solids | 10.2% |
| Actual Weight Gain | 2.7% |
| Coating Accountability | 117.6% |
| Coating Time (min) | 12 |

| **Calcium Tablets** | **1.7%** | **2.7%** |
|---|---|---|
| Disintegration Test (SGF TS) 1 hour | Passed | Passed |
| Disintegration Test (SIF TS) Rupture Time | Passed (< 10 min) | Passed (< 15 min) |
| Film Adhesion (scraped with ss spatula) | good | good |
| Film Flexibility | good | good |

| **Creatine Tablets** | **1.7%** | **2.7%** |
|---|---|---|
| Disintegration Test (SGF TS) 1 hour | Passed | Passed |
| Disintegration Test (SIF TS) Rupture Time | Failed (mushy core) | Failed (2 fail/1 pass) |
| Film Adhesion (scraped with ss spatula) | good | good |
| Film Flexibility | good | good |

| **Placebo Tablets** | **1.7%** | **2.7%** |
|---|---|---|
| Disintegration Test (SGF TS) 1 hour | Failed | Failed |
| Disintegration Test (SIF TS) Rupture Time | n/a | n/a |
| Film Adhesion (scraped with ss spatula) | good | good |
| Film Flexibility | good | good |

## Claims

1. An enteric coating composition comprising:
0.01% to 8% ammoniated shellac;
0.01% to 8% polymer selected from the group consisting of alginates and alginic acid; and
less than 15% solids;
wherein the total content of the resin in the composition selected from shellacs, plant resins and synthetic resins is 0.01% to 10% and the total content of non-resin polymer in the composition is 0.01% to 10%.

2. The enteric coating composition of claim 1, wherein the alginate comprises sodium alginate.

3. The enteric coating composition of claim 1, comprising 1 % to 7% ammoniated shellac, or 0.02% to 4% polymer selected from the group consisting of alginates and alginic acid.

4. The enteric coating composition of claim 1, further comprising a plasticizer comprising at least one of glycerin, acetylated monoglycerides, polysorbate 80, stearic acid, and a combination thereof, or wherein the composition has a pH of 6 to 8.

5. A pharmaceutical or nutraceutical comprising a pharmaceutical agent or nutraceutical agent coated with an enteric coating composition, the enteric coating composition comprising 0.01% to 8% resin and 0.01% to 8% alginate, wherein the resin is ammoniated shellac and the enteric coating composition comprises less than 15% solids.

6. The pharmaceutical or nutraceutical of claim 5, wherein the pharmaceutical or nutraceutical is in the form of a capsule, a tablet, or a softgel.

7. A method of making a product, the method comprising applying an enteric coating composition to a substrate to form an enteric coating thereon, the enteric coating composition comprising:
0.01% to 8% ammoniated shellac;
0.01% to 8% polymer selected from the group consisting of alginates and alginic acid; and
less than 15% solids;
wherein the total content of the resin in the composition selected from shellacs, plant resins and synthetic resins is 0.01% to 10% and the total content of non-resin polymer in the composition is 0.01% to 10%.

8. A method of claim 7, wherein the substrate is loaded into a coating pan and the substrate is coated with an enteric coating composition to form an enteric coating.

9. The method of claim 7 or 8, wherein the enteric coating passes the Disintegration Test, SGF TS 1 hour or wherein the enteric coating produces a result of less than 1 hour for Disintegration Test, SIF TS.

10. The method of claim 8, wherein the coating step comprises an inlet temperature of 40°C to 65°C, or an outlet temperature of 20°C to 50°C.

11. The method of claim 7 or 8, wherein the substrate comprises a pharmaceutical, a nutraceutical, fruit, vegetable, agricultural product, or an industrial product.

## Patentansprüche

1. Enterale Beschichtungszusammensetzung, umfassend:
0,01% bis 8% ammoniakalischen Schellack;
0,01% bis 8% Polymer, das ausgewählt ist aus der Gruppe bestehend aus Alginaten und Alginsäure; und
weniger als 15% Feststoffe;
wobei der Gesamtgehalt des Harzes in der Zusammensetzung, das ausgewählt ist aus Schellacken, pflanzlichen Harzen und synthetischen Harzen, 0,01% bis 10% beträgt und der Gesamtgehalt des nicht auf Harzbasis beruhenden Polymers in der Zusammensetzung 0,01 % bis 10% beträgt.

2. Enterale Beschichtungszusammensetzung nach Anspruch 1, wobei das Alginat Natriumalginat umfasst.

3. Enterale Beschichtungszusammensetzung nach Anspruch 1, umfassend 1% bis 7% ammoniakalischen Schellack oder 0,02% bis 4% Polymer, das ausgewählt ist aus der Gruppe bestehend aus Alginaten und Alginsäure.

4. Enterale Beschichtungszusammensetzung nach Anspruch 1, ferner umfassend einen Weichmacher mit mindestens einem der folgenden: Glycerin, acetylierter Monoglyceride, Polysorbat 80, Stearinsäure und einer Kombination davon, oder wobei die Zusammensetzung einen pH-Wert von 6 bis 8 hat.

5. Pharmazeutikum oder Nutrazeutikum, umfassend ein pharmazeutisches Mittel oder nutrazeutisches Mittel, das beschichtet ist mit einer enteralen Beschichtungszusammensetzung, wobei die enterale Beschichtungszusammensetzung 0,01% bis 8% Harz und 0,01% bis 8% Alginat aufweist, wobei das Harz ammoniakalischer Schellack ist und die enterale Beschichtungszusammensetzung weniger als 15% Feststoffe aufweist.

6. Pharmazeutikum oder Nutrazeutikum nach Anspruch 5, wobei das Pharmazeutikum oder Nutrazeutikum in der Form einer Kapsel, einer Tablette oder eines Softgels vorliegt.

7. Verfahren zur Herstellung eines Produktes, wobei das Verfahren die Anwendung einer enteralen Beschichtungszusammensetzung auf ein Substrat umfasst, um eine enterale Beschichtung darauf zu bilden, wobei die enterale Beschichtungszusammensetzung aufweist:
0,01% bis 8% ammoniakalischen Schellack;
0,01% bis 8% Polymer, das ausgewählt ist aus der Gruppe bestehend aus Alginaten und Alginsäure;und
weniger als 15% Feststoffe;
wobei der Gesamtgehalt des Harzes in der Zusammensetzung, das ausgewählt ist aus Schellacken, pflanzlichen Harzen und synthetischen Harzen, 0,01% bis 10% beträgt und der Gesamtgehalt des nicht auf Harzbasis beruhenden Polymers in der Zusammensetzung 0,01% bis 10% beträgt.

8. Verfahren nach Anspruch 7, wobei das Substrat in eine Beschichtungstrommel geladen wird und das Substrat mit einer enteralen Beschichtungszusammensetzung beschichtet wird, um eine enterale Beschichtung zu erzeugen.

9. Verfahren nach Anspruch 7 oder 8, wobei die enterale Beschichtung den Zerfallstest, SGF TS 1 Stunde besteht oder wobei die enterale Beschichtung ein Ergebnis von weniger als 1 Stunde für den Zerfallstest SIF TS ergibt.

10. Verfahren nach Anspruch 8, wobei der Schritt des Beschichtens eine Einlasstemperatur von 40° bis 65° C oder eine Austrittstemperatur von 20° bis 50° C umfasst.

11. Verfahren nach Anspruch 7 oder 8, wobei das Substrat ein Pharmazeutikum, ein Nutrazeutikum, Obst-, Gemüse-, landwirtschaftliches Produkt oder ein industrielles Produkt umfasst.

## Revendications

1. Composition d'enrobage entérique comprenant:
de 0,01% à 8% de shellac ammoniacale;
de 0,01% à 8% de polymère choisi dans le groupe constitué d'alginates et d'acide alginique; et
moins de 15% de solides;
dans laquelle la teneur totale de la résine dans la composition choisie parmi des shellacs, des résines de plantes et des résines synthétiques est de 0,01% à 10% et la teneur totale du polymère qui n'est pas la résine dans la composition est de 0,01% à 10%.

2. Composition d'enrobage entérique selon la revendication 1, dans laquelle l'alginate comprend l'alginate de sodium.

3. Composition d'enrobage entérique selon la revendication 1, comprenant de 1% à 7% de shellac ammoniacale ou de 0,02% à 4% de polymère choisi dans le groupe constitué d'alginates et d'acide alginique.

4. Composition d'enrobage entérique selon la revendication 1, comprenant en outre un plastifiant comprenant au moins un parmi la glycérine, des monoglycérides acétylés, le polysorbate 80, l'acide stéarique, et une combinaison de ceux-ci, ou dans laquelle la composition possède un pH de 6 à 8.

5. Produit pharmaceutique ou nutraceutique comprenant un agent pharmaceutique ou un agent nutraceutique enrobé avec une composition d'enrobage entérique, la composition d'enrobage entérique comprenant de 0,01% à 8% de résine et de 0,01% à 8% d'alginate, dans lequel la résine est une shellac ammoniacale et la composition d'enrobage entérique comprend moins de 15% de solides.

6. Produit pharmaceutique ou nutraceutique selon la revendication 5, où le produit pharmaceutique ou nutraceutique est sous la forme d'une capsule, d'un comprimé ou d'un gel mou.

7. Procédé pour la fabrication d'un produit, la méthode comprenant l'application d'une composition d'enrobage entérique sur un substrat pour former un enrobage entérique sur celui-ci, la composition d'enrobage entérique comprenant:
de 0,01% à 8% de shellac ammoniacale;
de 0,01% à 8% de polymère choisi dans le groupe constitué d'alginates et d'acide alginique; et
moins de 15% de solides;
dans laquelle la teneur totale de la résine dans la composition choisie parmi des shellacs, des résines de plantes et des résines synthétiques est de 0,01% à 10% et la teneur totale du polymère qui n'est pas la résine dans la composition est de 0,01 % à 10%.

8. Procédé selon la revendication 7, dans laquelle le substrat est chargé dans un bac d'enrobage et le substrat est enrobé avec une composition d'enrobage entérique pour former un enrobage entérique.

9. Procédé selon la revendication 7 ou 8, dans laquelle l'enrobage entérique réussit l'essai de désagrégation, SGF TS 1 heure ou dans laquelle l'enrobage entérique produit un résultat de moins de 1 heure pour l'essai de désagrégation, SIF TS.

10. Procédé selon la revendication 8, dans laquelle l'étape d'enrobage comprend une température d'entrée de 40°C à 65°C ou une température de sortie de 20°C à 50°C.

11. Procédé selon la revendication 7 ou 8, dans laquelle le substrat comprend un produit pharmaceutique, un produit nutraceutique, un fruit, un légume, un produit agricole ou un produit industriel.
